# EUROPEAN PATENT SPECIFICATION

(11) **EP 2 659 872 B1**
(45) Date of publication and mention of the grant of the patent: **21.03.2018**
(21) Application number: 11854187.9
(22) Date of filing: 26.12.2011
(51) Int. Cl.: A61F 13/496, A61F 13/15, A61F 13/49, A61F 13/494, A61F 13/53

(54) **UNDERWEAR-TYPE ABSORBENT ARTICLE**
SAUGFÄHIGE UNTERWÄSCHE
ARTICLE ABSORBANT DE TYPE SOUS-VÊTEMENT

(30) Priority: 27.12.2010 JP 2010291049
(43) Date of publication of application: 06.11.2013
(73) Proprietor: Kao Corporation, Tokyo 103-8210 (JP)
(72) Inventor: ONDA, Aiko, Haga-gun Tochigi 321-3497 (JP); SASAKI, Jun, Haga-gun Tochigi 321-3497 (JP)
(74) Representative: Vossius & Partner Patentanwälte Rechtsanwälte mbB
(86) International application number: PCT/JP2011/080027
(87) International publication number: WO 2012/090916

(56) References cited:
- WO-A1-2005/094746
- WO-A1-2011/001937
- JP-A- 2007 268 253
- JP-A- 2008 188 050
- JP-A- 2008 253 285
- JP-A- 2008 508 082
- US-A1- 2006 030 831

## Description

### Technical Field

The present invention relates to a pull-on absorbent article such as a disposable diaper.

### Background Art

Conventionally, there has been known a pull-on absorbent article including an outer cover shaped like a sandglass extended over a front portion to be worn about the front of a wearer, a crotch section to be worn about the crotch of a wearer, and a rear portion to be worn about the back of a wearer, and an absorbent assembly which is fixed to the inner face side of the outer cover. Here, both side edge portions of the outer cover at the front portion and both side edge portions of the outer cover at the rear portion are joined, so that a waist opening and a pair of leg openings are formed.

When continuously manufacturing such pull-on absorbent articles, it is common that penetrating holes or cutouts for forming leg openings are formed at a continuous precursor of the outer cover and that unnecessary portions are eliminated as being trimmings.

Further, as a conventional pull-on absorbent article, there has been known a pull-on absorbent article in which an outer cover is divided into a front panel to be worn about the front of a wearer and a rear panel to be worn about the back of a wearer, an absorbent assembly is fixed as bridging between the front panel and the rear panel, and both lateral side edge portions of the front panel and both lateral side edge potions of the rear panel are joined.

For example, Patent Literature 1 discloses a pull-on clothing article as a pull-on absorbent article which includes an absorbent assembly and a circular elastic belt structured with a front belt portion and a rear belt portion. Here, a length of the rear belt portion (rear panel) in the longitudinal direction is longer than a length of the front belt portion (front panel) in the longitudinal direction.

However, this type of a disposable diaper has a problem of being prone to provide an uneasy feeling for leakage with a vicinity of the groin portion at the front side and buttocks exposed.

Further, Patent Literature 2 discloses an open type disposable diaper in which, when being sectionalized into a front portion, a crotch section, and a rear portion, a low-basis-weight region is arranged at a portion of an absorbent assembly at the rear portion and a high-basis-weight region is arranged at a portion of the absorbent assembly at the crotch section. Here, at least one of a standing gather and a leg gather is not substantially formed at the rear portion where the low-basis-weight region is arranged.

### Citation List

### Patent Literature

Patent Literature 1 : JP 2008-508082 A
Patent Literature 2: JP 2007-268253 A

### Summary of Invention

With a disposable pull-on diaper of an outer cover front-rear separation type which is divided into a front panel to be worn about the front of a wearer and a rear panel to be worn about the back of a wearer, there is a problem that an uneasy feeling for leakage is prone to be provided with a vicinity of the groin portion at the front side and buttocks of a wearer exposed largely to the outside of both side edges of an absorbent assembly.

Further, since an extension portion does not exist at the front side, a problem in appearance is caused by insufficient covering of skin as a clothing article while worn.

On the other hand, when a width of the absorbent assembly is simply enlarged, a strange feeling or an uncomfortable feeling is prone to be provided at the groin or crotch while side portions of the absorbent assembly are strongly abutted to the inner side of the thighs.

The open type disposable diaper of Patent Literature 2 is for facilitating fitting of the diaper to the back of a wearer as enabling the rear portion to be planarly spread when wearing the diaper and is not for lessening a strange feeling or an uncomfortable feeling at the groin or crotch with a pull-on disposable diaper of an outer cover front-rear separation type.

The present invention relates to a pull-on absorbent article of an outer cover front-rear separation type which is less likely to provide a strange feeling and an uncomfortable feeling at the crotch of a wearer as well as an uneasy feeling for leakage.

The present invention provides a pull-on absorbent article having a waist opening and a pair of leg openings including a rectangular front panel adapted to be worn around a wearer's front, a rectangular rear panel adapted to be worn around a wearer's back, and an absorbent assembly fixed to the front panel and the rear panel so as to bridge them. Here, each of the front panel and the rear panel includes a body portion which includes side seals located at both lateral sides of the panels and an extension portion which is extended from the body portion toward a crotch section and which has no side seals at both lateral sides of the panels. The absorbent assembly includes an absorbent member oblong in longitudinal direction and side sheet portions located outward of both edges in the longitudinal direction of the absorbent member. The side sheet portion includes a stretchable region having stretchability due to an elastic member fixed to the side sheet portion in a stretched state, the stretchable region being located at the crotch section and on the front panel and the rear panel. The stretchable region is formed in such a manner that a length from a center line in the longitudinal direction of the article to an end part of the stretchable region located on the side of the front panel is larger than a length from the center line in the longitudinal direction of the article to an end part of the stretchable region located on the side of the rear panel. The entire range of a portion of the stretchable region located on the front panel is not joined onto the front panel and at least a part of the stretchable region located on the rear panel is joined onto the rear panel.

### Brief Description of Drawings

[FIG. 1] FIG. 1 is a perspective view illustrating a usage state (worn state) of a disposable pull-on diaper being an embodiment of the present invention.
[FIG. 2] FIG. 2 is a partially-sectioned plane view illustrating an opened and extended state of the disposable pull-on diaper illustrated in FIG. 1. Here, the opened and extended state denotes a state that joint portions (side seals) at both sides of a diaper are torn off to put a pull-on absorbent article into an opened state and that the absorbent article in the opened state is extended to have designed dimensions (same as dimensions when being planarly extended with influences of elastic members completely eliminated) with the respective elastic members extended.
[FIG. 3] FIG. 3 is a plane view (view corresponding to FIG. 2) illustrating another embodiment of a front portion of the disposable pull-on diaper illustrated in FIG. 1.
[FIG. 4] FIGs. 4(a) to 4(d) are plane views (views corresponding to FIG. 2) illustrating other embodiments of a rear portion of the disposable pull-on diaper illustrated in FIG. 1.
[FIG. 5] FIG. 5 is an enlarged sectional view at line III-III in FIG. 2.
[FIG. 6] FIG. 6 is an enlarged sectional view at line IV-IV in FIG. 2.
[FIG. 7] FIG. 7 is an enlarged sectional view at line V-V in FIG. 2.
[FIG. 8] FIG. 8 shows schematic sectional views of an absorbent assembly in a worn state of the disposable pull-on diaper illustrated in FIG. 1. FIG. 8(a) is a schematic sectional view at a portion at a slightly front side from a center part of the crotch of a wearer. FIG. 8(b) is a schematic sectional view at a portion at a slightly rear side from the center part of the crotch of the wearer.
[FIG. 9] FIG. 9 shows views illustrating a disposable pull-on diaper of a second embodiment of the present embodiment. FIG. 9(a) is a view of an absorbent assembly of the diaper in an opened and extended state viewing from a diaper inner face side. FIG. 9(b) is a schematic sectional view (in a natural state) at line Xa-Xa in FIG. 9(a).
[FIG. 10] FIG. 10(a) is a schematic sectional view specifically illustrating a laminated structure of a side sheet portion of the first embodiment. FIG. 10(b) is a schematic sectional view illustrating a modified example of the laminated structure of the side sheet portion.

### Description of Embodiments

In the following, a pull-on absorbent article of the present invention will be described based on preferable embodiments with reference to the drawings.

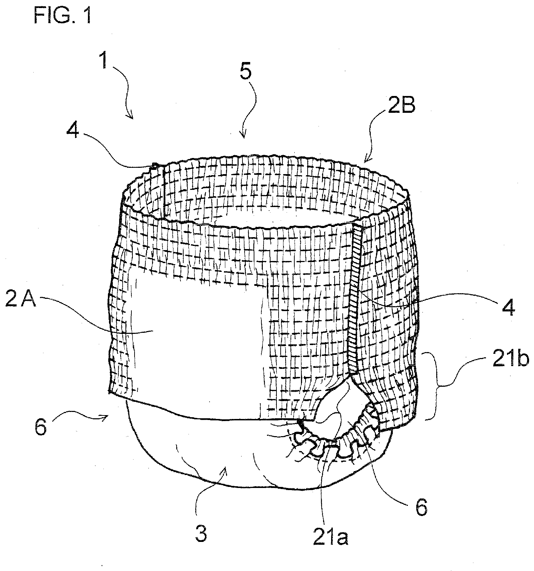

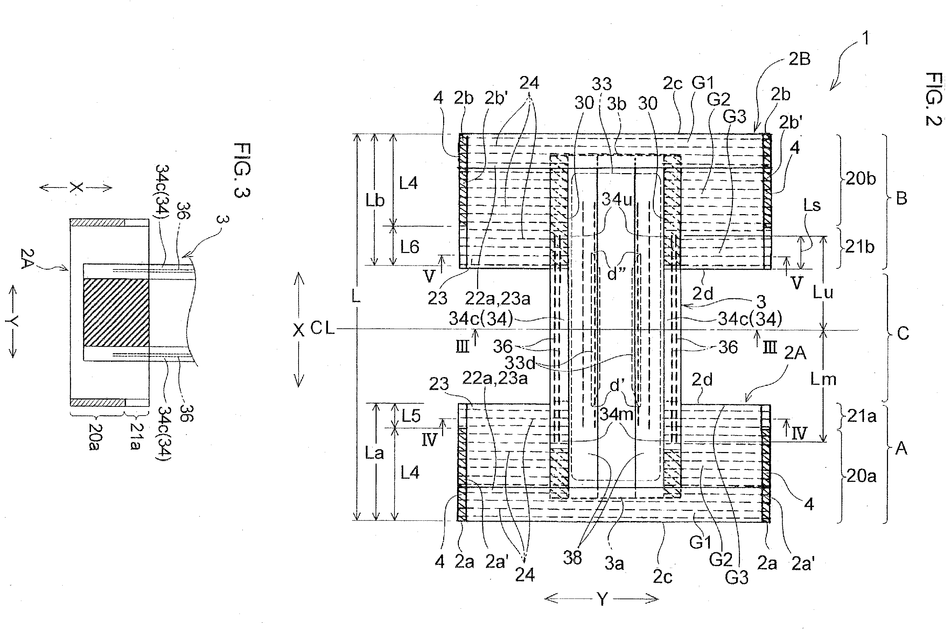

As illustrated in FIGs. 1 and 2, a disposable pull-on diaper 1 (hereinafter, called the diaper 1) of a first embodiment of the present invention includes a rectangular front panel 2A adapted to be worn about the front of a wearer, a rectangular rear panel 2B adapted to be worn about the back of a wearer, and an absorbent assembly 3 fixed to the front panel 2A and the rear panel 2B so as to bridge them. A pair of side seals 4, 4 is formed by joining both side edge portions 2a, 2a of the front panel 2A and side edge portions 2b, 2b of the rear panel 2B.

As illustrated in FIGs. 1 and 2, the diaper 1 includes a front portion A adapted to be worn around a wearer's, front, a rear portion B adapted to be worn around a wearer's back, and a crotch section C located between the front portion A and the rear portion B and adapted to be worn around a wearer's crotch. A diaper longitudinal direction (article longitudinal direction) denotes a direction from the front portion A to the back section B via the crotch section C or a direction opposite thereto (direction X in FIG. 2). A diaper lateral direction (article lateral direction) denotes a direction along the waist line of a wearer (direction Y in FIG. 2) and a direction perpendicular to the article longitudinal direction. In the following, the diaper longitudinal direction (article longitudinal direction) and the diaper lateral direction (article lateral direction) may be simply called direction X and direction Y respectively, as well.

The front panel 2A of the diaper 1 is rectangular shape oblong in the lateral direction in an opened and extended state of the diaper 1 (see FIG. 2) and includes the pair of lateral side edge portions 2a, 2a along the diaper longitudinal direction (direction X) and a pair of longitudinal edge portions 2c, 2d (the upper edge portion 2c and the lower edge portion 2d) along the diaper lateral direction (direction Y). Similarly, the rear panel 2B is rectangular shape oblong in the lateral direction in an opened and extended state of the diaper 1 (see FIG. 2) and includes the pair of lateral side edge portions 2b, 2b along direction X and a pair of longitudinal edge portions 2c, 2d (the upper edge portion 2c, the lower edge portion 2d) along the diaper lateral direction (direction Y). As illustrated in FIG. 2, lengths in the diaper longitudinal direction (direction X) of the front panel 2A and the rear panel 2B are even along the diaper lateral direction (direction Y).

The abovementioned pair of side seals 4, 4 is formed at the diaper 1 by joining the side edge portion 2a (specifically, a joint portion 2a' being a part thereof) of the front panel 2A and the side edge portion 2b (specifically, a joint portion 2b' being a part thereof) of the rear panel 2B. For example, known joining means such as heat sealing, high-frequency sealing, ultrasonic sealing, and adhesive is adopted for the joining. According to the joining, a waist opening 5 and a pair of leg openings 6, 6 are formed as well as the side seals 4, 4.

As illustrated in FIG. 2. in the side seal 4, a length L4 in direction X is smaller than both of a length La of the front panel 2A and a length Lb of the rear panel 2B. A body portion 20a, 20b having the side seals 4, 4 at both lateral sides and an extension portion 21a, 21b which is extended from the body portion 20a, 20b toward the crotch section C and which has no side seals 4, 4 at both lateral sides are formed at each of the front panel 2A and the rear panel 2B.

In the following, the body portion 20a and the extension portion 21a of the front panel 2A are also called the front body portion 20a and front extension portion 21a, respectively. The body portion 20b and the extension portion 21b of the rear panel are also called the rear body portion 20b and the rear extension portion 21b. respectively.

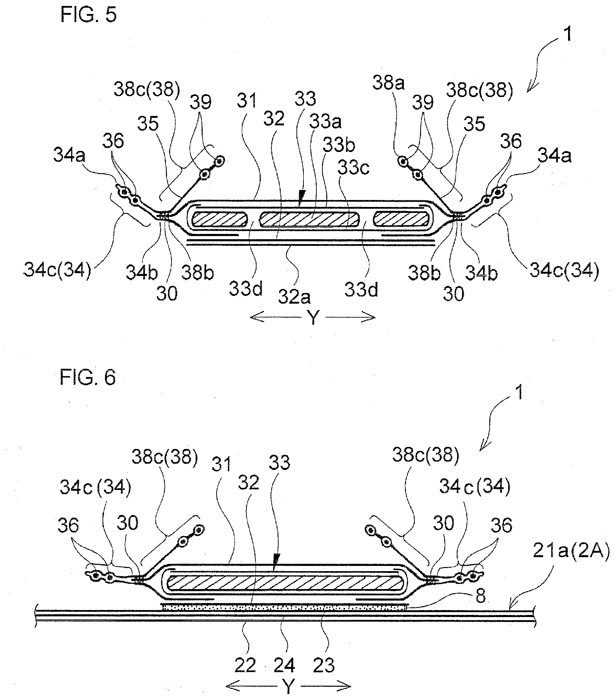

The absorbent assembly 3 of the diaper 1 is provided with a liquid permeable topsheet 31, a liquid impermeable or water-repellent backsheet 32, and a liquid retaining absorbent member 33 interposed between both the sheets 31, 32 as illustrated in FIG. 5 and is formed into a rectangular shape oblong in direction X as illustrated in FIG. 2. As illustrated in FIG. 5, the absorbent member 33 includes an absorbent core 33a containing an aggregate of fibers (may be nonwoven as well) such as pulp fibers or an aggregate of fibers and particles of an absorbent polymer retained in the fibers and core wrap sheets 33b, 33c which cover the absorbent core 33a. The absorbent member 33 is formed into a rectangular shape being long in direction X as well. The absorbent member 33 and the backsheet 32 are joined with arbitrary joining means such as adhesive, heat sealing, and ultrasonic sealing.

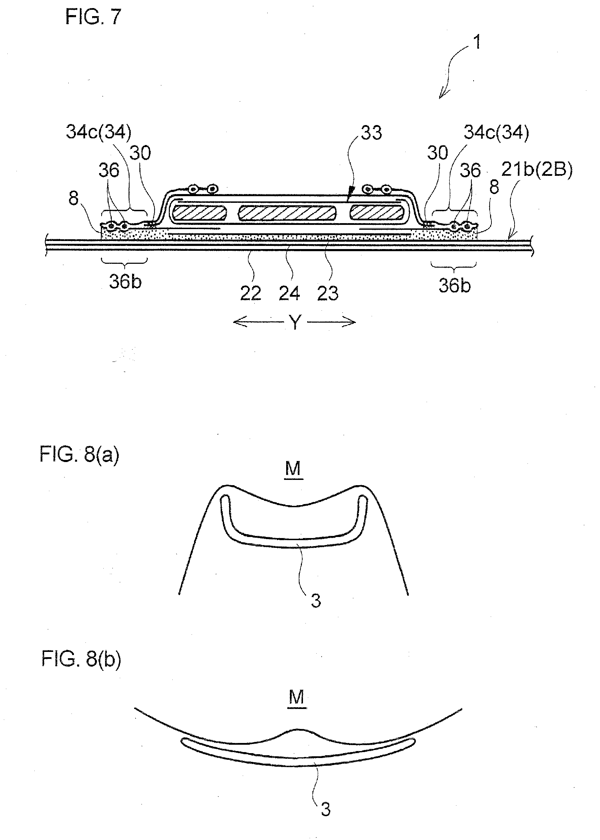

One end side in the longitudinal direction (a portion overlapped with the front panel 2A) of the absorbent assembly 3 is fixed to a center region in direction Y of the front panel 2A with adhesive 8, as illustrated in FIG. 6. The other end side in the longitudinal direction (a portion overlapped with the rear panel 2B) thereof is fixed to a center region in direction Y of the rear panel 2B with adhesive 8, as illustrated in FIG. 7.

The absorbent assembly 3 includes, at both side portions in the longitudinal direction, side sheet portions 34, 34 which are located outward of both side edges in the longitudinal direction of the absorbent member 33.

As illustrated in FIG. 2, the side sheet portion 34 is extended along the longitudinal direction of the absorbent assembly 3 and exists over the entire length of the absorbent assembly 3. As illustrated in FIG. 5, the side sheet portion 34 includes a liquid-resistant or water-repellent sheet material 35 and elastic members 36, 36 fixed to the sheet material 35.

A range of the side sheet portion 34 where the elastic member 36 is arranged in the longitudinal direction is a stretchable region 34c where the elastic member is arranged in a stretched state in the longitudinal direction of the absorbent assembly. The stretchable region 34c is stretchable in the longitudinal direction of the absorbent assembly 3.

The stretchable region 34c of the side sheet portion 34 forms a side gather at the crotch section C (between the front panel 2A and the rear panel 2B in direction X of the absorbent assembly 3).

That is, the stretchable region 34c at the crotch section C forms the side gather with contraction of the elastic member 36 which is arranged at the side sheet portion 34 in a stretched state while the sheet material 35 structuring the side sheet portion 34 is gathered or deformed into a wavelike sectional shape. The side gather is formed at the crotch section C of the diaper 1 at least in a natural state and/or a worn state and is abutted to skin of a wearer as being raised toward the skin of the wearer.

As illustrated in FIG. 2, the stretchable region 34c of the side sheet portion 34 of the diaper 1 includes one end portion 34m (hereinafter, also called the front end portion 34m) in the front panel 2A. Further, as illustrated in FIG. 2, the stretchable region 34c includes the other end portion 34u (hereinafter, also called the rear end portion 34u) on the side of the rear panel 2B. Then, as illustrated in FIG. 2, in the stretchable region 34c, a length Lm from a center line CL in the longitudinal direction of the diaper 1 to the front end portion 34m is larger than a length Lu from the center line CL in the longitudinal direction to the rear end portion 34u.

A width of the crotch of a wearer is narrowed at the front side (belly side) from a center part of the wearer's crotch where the center line CL in the longitudinal direction is located (see FIG. 8(a)). Accordingly, when the absorbent assembly 3 to be worn about the portion is wide, both side portions of the absorbent assembly 3 press the vicinity of inner sides of the thighs of a wearer M and a strange feeling or an uncomfortable feeling is prone to be provided to the wearer. However, when the length Lm of a portion located at the front side from the center line CL in the longitudinal direction of the stretchable region 34c is set long, the stretchable region 34c of the side sheet portion 34 at the front side exists in a broad range in the longitudinal direction of the side sheet portion 34 at the front side. Accordingly, the side sheet portion 34 at the front side is more likely to be raised in a direction being close to skin of the wearer M. Further, as illustrated in FIG. 8(a), both the side portions of the absorbent assembly 3 are more likely to be raised in a direction being close to the skin of the wearer M as following to the side sheet portion 34. Since the absorbent assembly 3 is narrowed when the side sheet portion 34 and the absorbent assembly 3 are raised, a strange feeling or an uncomfortable feeling is less prone to be provided to the wearer. In addition, fit at the crotch section is improved and clearance is less likely to be caused even in a state that clearance is prone to be caused at the crotch section during action, sitting, or the like. Accordingly, excreta can be prevented from being leaked.

On the other hand, at the rear side from the center part of the wearer's crotch where the center line CL in the longitudinal direction is located, there is less influence of constriction of both legs and buttocks are shaped convexly outward. Accordingly, a width of the wearer's crotch is larger compared to the front side (see FIG. 8(b)). In a case that the absorbent assembly 3 is narrowed with the side sheet portion 34 raised as the abovementioned front side, buttocks are to be largely exposed and look like being protruded. Accordingly, the wearer is caused to have an uneasy feeling for liquid-leakage in appearance as well as a strange feeling. Particularly, when feces are loose and spread toward the buttocks, the feces are prone to stick to a standing gather or the side sheet portion. There may be a case that a clothing article gets wet depending on actions. Due to that the length Lu of the portion located at the rear side from the center line CL in the longitudinal direction at the stretchable region 34c is set short, the side sheet portion 34 and the absorbent assembly 3 are more likely to be planarly fitted in a broad range to a curved shape of skin of the wearer M without being excessively raised, as illustrated in FIG. 8(b). Accordingly, biting of both side portions of the absorbent assembly 3 and side sheet portion 34 to skin is less likely to occur and fit to the buttocks and appearance are improved. In addition, liquid leakage from the buttocks can be prevented.

From a viewpoint to reliably obtain the abovementioned effects, the length Lm of the portion located at the front side from the center line CL in the longitudinal direction of the stretchable region 34c and the length Lu from the center line CL in the longitudinal direction to the rear end portion 34u satisfy "1.1Lu ≤ Lm ≤ 4.0Lu" preferably, and "1.1Lu ≤ Lm ≤ 3.0Lu" more preferably.

From the same viewpoint, the length Lm is in a rage of 52% to 80% of the total length (Lm + Lu) of the stretchable region 34c preferably, in a range of 55% to 75% more preferably, and in a range of 55% to 70% even more preferably.

Further, the length Lm is in a range of 25% to 40% of the total length L of the diaper 1 preferably, and in a range of 28% to 38% more preferably. The length Lu is in a range of 10% to 35% of the total length L of the diaper 1 preferably, and in a range of 15% to 30% more preferably.

Here, when the length Lm is enlarged and both side portions of the absorbent assembly 3 at the front side portion of the crotch of a wearer are raised, a strange feeling at the crotch section of the wearer is lessened. However, the vicinity of the groin at the front side of the wearer is exposed laterally from the narrowed absorbent assembly 3 and appearance thereof is prone to provide an uneasy feeling for leakage. Here, according to the diaper 1 of the present embodiment, since the front extension portion 21a can cover the vicinity of the groin of the wearer as being extended laterally from the absorbent assembly 3, the appearance can be prevented from being worsened.

As illustrated in FIG. 2, in the diaper 1, the stretchable region 34c of the side sheet portion 34 is extended onto the front panel 2A as well. As illustrated in FIGs. 2 and 6, the entire range of a portion of the stretchable region 34c located on the front panel 2A is not joined onto the front panel 2A. The side sheet portion 34 is joined onto the front panel 2A and the rear panel 2B at positions being close to end parts 3a, 3b in the longitudinal direction X of the absorbent assembly 3 from the front end portion 34m and the back end portion 34u of the stretchable region 34c. Shaded ranges of the side sheet portion 34 in FIG. 2 are ranges joined to the front panel 2A and the rear panel 2B with adhesive 8. Here, the entire range denotes the entire range in direction X. It is determined whether or not the entire range of the stretchable region 34c is joined by whether or not a range where the elastic member 36 is placed and a range outside therefrom of the side sheet portion 34 in direction Y are joined to the front panel 2A over the entire range in direction X.

The absorbent assembly 3 is joined to the front panel 2A over the entire range in direction X in a range of 40% to 100% of the length of the absorbent assembly 3 in direction Y preferably, and in a range of 55% to 95% more preferably. In this case, joining over the entire range in direction X also includes a case that a non-joint portion with a width of 10 mm or less where the absorbent assembly 3 and the front panel 2A are not joined exists at the lower edge portion 2d of the front panel 2A.

The shaded range of the side sheet portion 34 in FIG. 2 is fixed to the front panel 2A or the rear panel 2B in a state of being laid outward in the lateral direction (direction Y) of the diaper 1.

Meanwhile, as illustrated in FIG. 2, the stretchable region 34c of the side sheet portion 34 is extended onto the rear panel 2B as well. As illustrated in FIGs. 2 and 7, at least a part of the stretchable region 34c located at the rear panel 2B is joined onto the rear panel 2B. It is determined whether or not a part of the stretchable region 34c is joined also by whether or not a range where the elastic member 36 is placed and a range outside therefrom of the side sheet portion 34 in direction Y are joined to the rear panel 2B at a part in direction X.

Owing to that the entire range of the stretchable region 34c located on the front panel 2A is not joined to the front panel 2A, rising characteristics of both side portions of the absorbent assembly 3 are improved and a strange feeling or an uncomfortable feeling is further prevented from occurring at the front side portion of the crotch of a wearer. Owing to that at least a part of the portion located on the rear panel 2B is joined to the rear panel 2B, the absorbent assembly 3 is abutted to a range from a portion of the crotch of the wearer at the rear side to a portion of the buttocks in a state that the absorbent assembly 3 is expanded in the lateral direction. Accordingly, fitting to the portions and covering of the buttocks are further improved. From viewpoints of the fitting and covering, it is preferable that the entire range or a part of the stretchable region 34c is joined onto the rear extension portion 21b of the rear panel 2B.

When a length in direction X of overlapping between the rear panel 2B and the stretchable region 34c is denoted by Ls, the joint portion where the stretchable region 34c is joined to the rear panel 2B is in a range of 10% to 95% of Ls from a waist edge portion side at the rear panel 2B preferably, and in a range of 30% to 95% more preferably. Joining over the entire range in direction X also includes a case that a non-joint portion with a width of 10 mm or less where the absorbent assembly 3 and the rear panel 2B are not joined exists at the lower edge portion 2d of the rear panel 2B. The absorbent assembly 3 is joined to the rear panel 2B over the entire range in direction X in a range of 40% to 100% of the length of the absorbent assembly 3 in direction Y preferably, and in a range of 55% to 95% more preferably.

Further, as illustrated in FIG. 2, in the diaper 1, the stretchable region 34c reaches the front body portion 20a as traversing the front extension portion 21a of the front panel 2A and the front end portion 34m is located on the front body portion 20a. In contrast, the rear end portion 34u of the stretchable region 34c is located on the rear extension portion 21b without reaching the rear body portion 20b. According to the above structure, the abovementioned effects due to that the length Lm is set larger than the length Lu can be obtained more effectively.

The joint position between the side sheet portion 34 and the front panel 2A is not limited to the embodiment illustrated in FIG. 2. For example, as illustrated in FIG. 3, the side sheet portion 34 including the stretchable region 34c located on the front panel 2A may be in a non-joined state to the front panel 2A over the entire range in direction X and over the entire range in direction Y. The front panel 2A and the absorbent assembly 3 are in a joined state at a shaded part in the drawing. Owing to joining the front panel 2A and the absorbent assembly 3 as described above, rising characteristics of the stretchable region 34c are improved at the front portion A and fitting between the stretchable region 34c and the wearer's body is improved. Accordingly, clearance can be effectively prevented from occurring between the stretchable region 34c and the wearer's body. In addition, a strange feeling can be effectively prevented from occurring around the wearer's legs. Here, the elastic members 24 are not illustrated in FIG. 3.

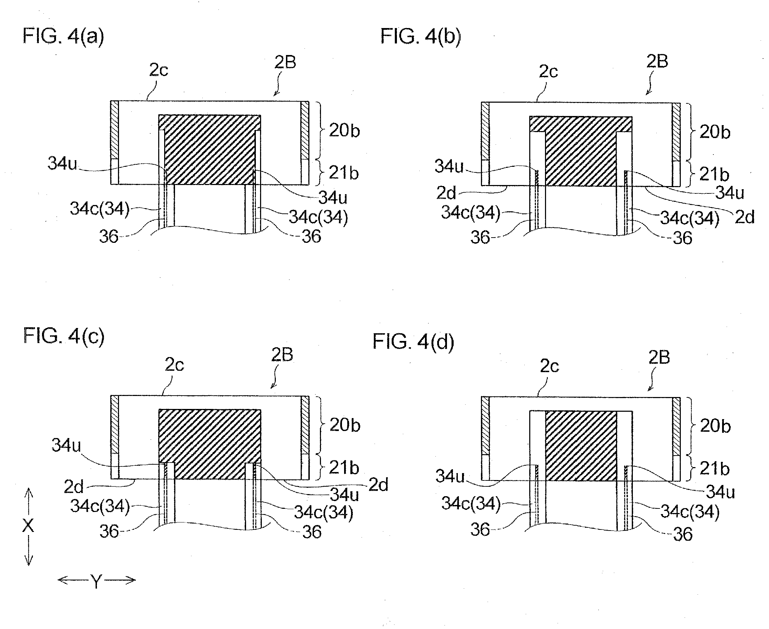

Similarly, the joint position between the portion of the stretchable region 34c located on the rear panel 2B and the rear panel 2B is not limited to the embodiment illustrated in FIG. 2. For example, as illustrated in FIG. 4(a), the portion of the stretchable region 34c located on the rear panel 2B is joined to the rear panel 2B at the rear extension portion 21b over the entire range in direction X. Due to joining as described above, there arises an advantage that covering of the wearer's buttocks with the rear panel 2B is improved, Regarding direction Y, the stretchable region 34c may be in a non-joined state to the rear panel 2B at the outer side from the elastic member 36 in direction Y while being joined to the rear panel 2B only at the inner side from the elastic member 36 in direction Y. Owing to performing joining as described above, there arises an advantage that air permeability of the stretchable region 34c is improved at a part being outer side from the elastic member 36 in direction Y. Here, in FIG. 4(a), the rear panel 2B and the absorbent assembly 3 are in a joined state at a shaded part and the elastic members 24 are not illustrated (being the same in FIGs. 4(b) to 4(d) as well which will be described in the following).

It is also possible to adopt joining illustrated in FIGs. 4(b) to 4(d) for targeting to further improve air permeability at a part of the stretchable region 34c located on the rear panel 2B. In FIGs. 4(b) to 4(d), regarding direction Y, the stretchable region 34c is joined to the rear panel 2B only at a part where the elastic member 36 is arranged. Here, the stretchable region 34c is in a non-joined state to the rear panel 2B at a part being outer side and a part being inner side from the elastic member 36 in direction Y. Meanwhile, regarding direction X, it is as described below.

In FIG. 4(b), the side sheet portion 34 is in a non-joined state to the rear panel 2B at the rear extension portion 21a. Further, the side sheet portion 34 is in a joined state to the rear panel 2B at a part of the rear body portion 20b being close to the upper edge portion 2c and in a non-joined state to the rear panel 2B at a part of the rear body portion 20b being close to the lower edge portion 2d. In FIG. 4(c), the side sheet portion 34 is in a joined state to the rear panel 2B over the entire range of the rear body portion 20b. At the rear extension portion 21a, a part being close to the upper edge portion 2c is in a joined state to the rear panel 2B and a part being closed to the down edge portion 2d is in a non-joined state to the rear panel 2B. In FIG. 4(d), the side sheet portion 34 is in non-joined state to the rear panel 2B over entire ranges of both of the rear body portion 20b and the rear extension portion 21a. Here, similarly to FIG. 4(a), the rear end portion 34u is located in the rear extension portion 21a in FIGs. 4(b) to 4(c).

As illustrated in FIGs. 2 and 5, the absorbent member 33 includes, at both sides in the longitudinal direction, low stiffness portions 33d without having a forming material of the absorbent core 33a or with a basis weight of the forming material being smaller than that of another part. The low stiffness portions 33d are formed as being extended along the longitudinal direction of the absorbent member at positions being apart from both side edges of the absorbent member 33. Since the absorbent member 33 is easy to be bent at the low stiffness portions 33d, both the side portions of the absorbent assembly 3 are more likely to be raised toward wearer's skin when wearing the diaper. Examples of the forming material of the absorbent core 33a include fiber materials such as pulp fibers and absorbent polymer.

Further, as illustrated in FIG. 2, in the stretchable region 34c of the side sheet portion 34, the front end portion 34m in the longitudinal direction is extended from a position of an end part d' of the low stiffness portion on the side of the front panel 2A toward an edge 2c the waist opening of the front panel 2A.

According to the above structure, when the stretchable region 34c at the front side is contracted, the contracting region 34c can be contracted so as to enwrap a portion of the absorbent core 33 located at the outer side from the low stiffness portion 33d being a portion raised as being bent at the low stiffness portion 33d. Accordingly, a strange feeling at the crotch section can be lessened by further narrowing the crotch section of the absorbent assembly 3. The length Lm in direction X of the stretchable region 34c from the center line CL in direction X of the diaper 1 to the front end portion 34m is in a range of 1.2 to 2.5 times of the length in direction X from the center line CL in direction X of the diaper 1 to the end part d' in direction X of the low stiffness portion 33d preferably, and in a range of 1.3 to 2.0 times thereof more preferably.

As illustrated in FIG. 2, in the stretchable region 34c of the side sheet portion 34, the rear end portion 34u in the longitudinal direction is also extended from a position of an end part d" of the low stiffness portion 33d on the side of the rear panel 2B toward the edge 2c of the waist opening of the rear panel 2B. According to the above structure, the absorbent member is more likely to be raised at the crotch section sandwiched by the two legs and a strange feeling at the crotch section can be lessened. Further, due to that the length Lu is smaller than the length Lm while the end part d" of the low stiffness portion 33d on the side of the rear panel 2B is located to the crotch side from the rear end portion 34u in the longitudinal direction of the stretchable region 34c, rising of the absorbent member can be suppressed at the buttocks at the rear side. Accordingly, the buttocks can be further prevented from being protruded. The length Lu in direction X of the stretchable region 34c from the center line CL in direction X of the diaper 1 to the rear end portion 34u is in a range of 1 to 2.5 times of the length in direction X from the center line CL in direction X of the diaper 1 to the end part d" in direction X of the low stiffness portion 33d preferably, and in a range of 1 to 2.0 times thereof more preferably.

Here, the edge 2c of the waist opening is the edge portion (upper edge portion) 2c which forms an opening circumferential edge of the waist opening at each of the front panel any the rear panel 2B.

As illustrated in FIG. 2, the diaper 1 includes a standing gather forming portion 38 which is located laterally inward of the side sheet portion 34 in the both lateral sides of the absorbent assembly 3 and which is extended along the longitudinal direction of the absorbent assembly 3.

As illustrated in FIG. 5, the standing gather forming portion 38 includes a sheet material 35 and an elastic member 39 for forming a standing gather fixed to the sheet material 35. The standing gather forming portion 38 forms a standing gather 38c at least at the crotch section C. The sheet material 35 in the diaper 1A of the present embodiment is a laminated sheet having a two-layer structure obtained by two-folding one sheet along a folding part arranged at an end part 38a at a free end side of the standing gather 38c. The elastic member 39 is fixed between the layers of the laminated sheet with adhesive. Further, the laminated sheet is further two-folded along a folding part arranged at an end part 34a at a free end side of the side gather at the side sheet portion 34, so that the elastic member 36 is fixed between the mutually-faced two laminated sheets (four sheet members 35) with adhesive. Further, the laminated sheets are joined and integrated in a state that the topsheet 31 is interposed therebetween at a linear joint portion 30 formed at a vicinity of the side edge portion of the absorbent member 33, more specifically, at a position being slightly outward of the side edge portion.

As illustrated in FIG. 2, the linear joint portion 30 is extended along each of both side edge portions of the rectangular absorbent member 33 and is extended along the entire length in direction X of the absorbent member 33 and the absorbent assembly 3. It is preferable that the linear joint portion 30 is formed into a continuously linear shape. However, it is also possible to be formed into a broken line shape. Further, a variety of joint methods such as heat sealing, ultrasonic sealing, high-frequency sealing, and adhesive may be adopted as a joint method for forming the linear joint portion 30.

The standing gather 38c is raised toward wearer's skin when wearing the diaper 1A and prevents liquid from leaking outward in the lateral direction from the absorbent assembly 3. The topsheet 31, the backsheet 32, the absorbent core 33a, and the core wrap sheets 33b, 33c may be made of materials respectively being the same which are conventionally used for such kinds of absorbent articles. From viewpoints of improving texture and the like, it is also possible to arrange an external sheet 32a made of nonwoven or the like on the side of the backsheet 32 of the absorbent assembly 3.

As illustrated in FIG. 2, on the side of the rear panel 2B in direction X of the diaper 1, the standing gather 38c of the diaper 1 is extended from the rear end portion 34u of the stretchable region 34c of the side sheet portion 34 toward the waist opening edge 2c of the rear panel 2B. Owing to that the length Lm is larger than the length Lu, the lateral protrusion of the buttocks is less likely to be a problem at the wearer's buttock side. Accordingly, on the side of the rear panel 2B, leakage prevention can be further improved without decreasing performance of covering buttocks and the like by forming the standing gather 38c long. The length in direction X from the center line CL of the diaper 1 to the end part at the rear side of a stretchable region of the standing gather 38c is in a range of 1.1 to 2.5 times of the length Lu in direction X from the center line CL of the diaper 1 to the rear end portion 34u of the side sheet portion 34 preferably, and in a range of 1.2 to 2.0 times thereof more preferably.

Meanwhile, as illustrated in FIG. 2, on the side of the front panel 2A in direction X of the diaper 1, the standing gather 38c of the diaper 1 does not reach the front end portion 34m of the stretchable region 34c of the side sheet portion 34. Accordingly, a strange feeling can be prevented from occurring at a part of the crotch being close to the front side while maintaining excellent leakage prevention performance with the standing gather 38c formed at the crotch section. The length in direction X from the center line CL of the diaper 1 to the end part at the front side of the stretchable region of the standing gather 38c is in a range of 0.5 time or more and less than 1.0 time of the length Lm in direction X from the center line CL of the diaper 1 to the front end portion 34m of the side sheet portion 34 preferably, and in a range of 0.7 time or more and less than 1.0 time thereof more preferably.

Both ends of the standing gather 38c in the longitudinal direction (direction X) denotes both ends of a portion of the standing gather forming portion 38 capable of being raised. In the embodiment illustrated in FIG. 2, a range where the elastic member 39 provides stretchability and a range where the standing gather forming portion 38 is capable of being raised (a range where the standing gather is formed) are the same.

Here, both end portions and the vicinities thereof in the longitudinal direction of the standing gather forming portion 38 are fixed onto the topsheet 31 in a state of being laid inward in the lateral direction (direction Y) of the diaper 1.

Further, from viewpoints that a strange feeling at the crotch is lessened with narrowed absorbent assembly 3 at the time of rising by improving rising performance of both side portions of the absorbent assembly 3 at the front side as locating the low stiffness portion 33d to the front side and the like, it is preferable that a center line in the longitudinal direction of the absorbent core 33a (a line extended in direction Y bisecting the entire length in direction X of the absorbent core) exists on the side of the front panel from the center line CL in the longitudinal direction of the article.

The length from the center line CL in the longitudinal direction of the article to the end part d' at the front side of the low stiffness portion 33d is in a range of 1.1 to 1.5 times of the length from the center line CL in the longitudinal direction of the article to the end part d" at the rear side of the low stiffness portion 33d preferably, and in a range of 1.1 to 2.0 times thereof more preferably. According to the above, it is possible to obtain effects to prevent biting and protruding of the buttocks with the rear side being more likely to follow curvature of the buttocks in addition to the effect to lessen a strange feeling at the crotch as improving rising characteristics of the absorbent assembly 3 at the crotch section. Further, owing to that the length in the longitudinal direction from the center line CL in the longitudinal direction of the article to the rear end portion of the absorbent core 33a is set larger than the length in the longitudinal direction from the center line CL in the longitudinal direction of the article to the front end portion of the absorbent core 33a, absorbing capacity at the front side can be increased. According to the above, a risk of possible leakage occurring due to narrowed crotch caused by improving easiness of being raised can be set off with absorbing capability increase at the front side. Accordingly, it is also possible to obtain an effect to prevent leakage.

As illustrated in Fig. 2, 6 and 7, each of the front panel 2A and the rear panel 2B of the diaper 1 includes an outer layer sheet 22 which forms a diaper outer face, an inner layer sheet 23 arranged at the inner face side of the outer layer sheet 22, and a plurality of thread-like elastic members 24 arranged between both the sheets 22, 23. A waist stretchable portion G1 and a torso stretchable portion G2 are formed at each of the front body portion 20a and rear body portion 20b. Further, an extension stretchable portion G3 is formed at each of the front extension portion 21a and the rear extension portion 21b.

At each of the front panel 2A and the rear panel 2B, the waist stretchable portion G1 is formed outward of the longitudinal end part 3a, 3b of the absorbent assembly 3 in the longitudinal direction (direction X) of the diaper 1. At each of the front panel 2A and the rear panel 2B, the torso stretchable portion G2 is formed between the waist stretchable portion G1 and the front extension portion 21a or the rear extension portion 21b in direction X. The extension stretchable portion G3 is formed at the front extension portion 21a or the rear extension portion 21b. The torso stretchable portion G2 and the extension stretchable portion G3 are formed at least at positions outward of both longitudinal edges of the absorbent assembly 3 in the lateral direction (direction Y) of diaper 1.

It is preferable that the waist stretchable portion G1, the torso stretchable portion G2, and the extension stretchable portion G3 provide stretchability at least at positions outward of both edges of the absorbent member 33 in the lateral direction (direction Y) of the diaper 1.

FIGs. 5 to 7 illustrate an example in which the elastic members 24 which form the waist stretchable portion G1, the torso stretchable portion G2, and the extension stretchable portion G3 are extended over the entire width of the front panel 2A and the rear panel 2B as traversing the absorbent member 33. Here, it is preferable that the elastic members 24 which form the torso stretchable portion G2 and/or the extension stretchable portion G3 do not provide stretchability to the front panel 2A and the rear panel 2B at portions where the elastic members 24 are overlapped with the center part in the lateral direction of the absorbent member 33 by eliminating elasticity of the elastic members with thermal treatment or the like, cutting the elastic members, or the like.

The two sheets which structure each of the front panel 2A and the rear panel 2B may be substantially fixed with adhesive over the entire range of the front panel 2A and the rear panel 2B or may be joined at a number of joint portions formed separately so as not to pass through the elastic members 24 as two sheet materials of an elastic stretchable member disclosed in JP 2005-80859 A.

In the front panel 2A, it is preferable that a plurality of elastic members 24 is arranged at each of the front body portion 20a and the front extension portions 21a at intervals in direction X. Similarly, in the rear panel 2B, it is also preferable that a plurality of elastic members 24 is arranged at each of the rear body portion 20b and the rear extension portion 21b at intervals in direction X.

A length L5 (see FIG. 2) of the front extension portion 21 a is in a range of 5% to 60% of a length La (see FIG. 2) of the front panel 2A preferably, and in a range of 20% to 40% thereof more preferably. A length L6 (see FIG. 2) of the rear extension portion 21b is in a range of 5% to 60% of a length Lb (see FIG. 2) of the rear panel 2B preferably, and in a range of 20% to 40% thereof more preferably. In a case of a diaper for an infant, the lengths L5, L6 of the front and rear extension portions 21a, 21b are in a range of 10 to 150 mm preferably, and in a range of 20 to 100 mm more preferably. In a case of a diaper for an adult, the lengths L5, L6 of the front and rear extension portions 21a, 21b are in a range of 10 to 200 mm preferably, and in a range of 20 to 150 mm more preferably.

From a viewpoint not to provide an impression that clearance between the front extension portion 21a and the rear extension portion 21b is caused by peeling at the side seal or splitting of the sheet, it is preferable that the length L6 of the rear extension portion 21b is longer than the length L5 of the front extension portion a (L6 > L5). Here, from viewpoints of improving worn appearance such as fitting to a constricted and curved body shape and preventing protrusion of the buttocks and improving a strange feeling while worn, the length L6 of the rear extension portion 21b is in a range of 1.2 to 10 times of the length L5 of the front extension portion 21a preferably, and in a range of 1.5 to 6.5 times thereof more preferably.

The lengths L5, L6 of the front extension portion 21 and the rear extension portion 21b denote lengths in the diaper longitudinal direction (direction X).

Here, as illustrated in FIG. 2, the outer layer sheet 22 and the inner layer sheet 23 of the diaper 1 of the abovementioned embodiment include folded extension portions 22a, 23a which are folded back to the inner layer sheet 23 side at the edge portion 2c which forms the opening circumferential end of the waist opening. The folded extension portions 22a, 23a are joined, at the side seal 4, to portions of the outer layer sheet 22 and the inner layer sheet 23 which are not folded back. Further, portions thereof overlapped with the absorbent assembly 3 in the longitudinal direction are joined to a surface of the absorbent assembly 3 on the side of the topsheet 31 with adhesive.

Although a variety of sheet materials which is conventionally used for such articles may be used as the outer layer sheet 22 and the inner layer sheet 23 without specific limitation, nonwoven is preferable. Particularly, from viewpoints of flexibility and the like, it is preferable to adopt nonwoven with a single layer or layered nonwoven with two or more layers made of air-through nonwoven, heat-roll nonwoven, spanlaced nonwoven, spunbond nonwoven, meltblown nonwoven, or the like. Further, it is also possible to adopt a sheet integrating the nonwoven with a film. A variety of known elastic materials which is used for absorbent articles such as disposable diapers and sanitary pads may be used as materials for forming the elastic members 24, 36, 39 without specific limitation. Examples of elastic materials include synthetic rubber such as styrene-butadiene, butadiene, isoprene, and neoprene, natural rubber, EVA. stretchable polyolefin, and polyurethane. The elastic members may be preferably thread-shaped (thread rubber or the like) with a section being rectangular, square, circular, polygonal or the like, string-shaped (flat rubber or the like), thread-shaped to be a multifilament type, or the like.

For example, a continuous precursor for the front panel 2A and a continuous precursor for the rear panel 2B are conveyed as being spaced and the absorbent bodies 3 are intermittently fixed to them so as to bridge them. Subsequently, heat sealing or the like (joining) for forming the side seals 4 is performed after two-folding causing the continuous precursor for the front panel 2A and the continuous precursor for the rear panel 2B to be overlapped. At the same time with or after the joining, cutting is performed to divide the above into an individual disposable diaper. In this manner, the abovementioned diaper 1 can be manufactured effectively. Compared to a case to form penetrating holes or cutouts for forming leg openings, according to this method, trimming of an precursor of an outer cover can be eliminated or portions to be trimmed can be lessened. Particularly, cutting the precursors for the front panel 2A and the rear panel 2B on a line perpendicular to a conveyance direction to form the front panel 2A and the rear panel 2B of the completed diaper 1 is preferable because the trimming can be unnecessary.

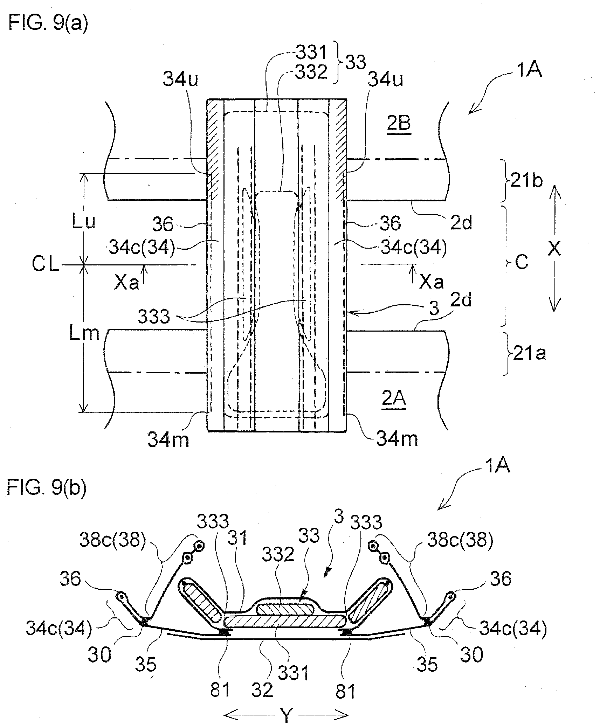

Next, a disposable pull-on diaper of a second embodiment of the present invention will be described with reference to FIG. 9.

Regarding the disposable pull-on diaper of the second embodiment, description will be performed mainly on different points from the first embodiment and description will not be repeated on the same points. Points which are not specifically described are the same as the first embodiment. In FIG. 9, the same numeral is given to a structural element or the like which is similar to a structural element or the like of the diaper of the first embodiment.

In the diaper 1A of the second embodiment, the topsheet 31 is rolled down to the back surface side of the absorbent member 33. Joint points 81 where the rolled-down portion of the topsheet 31 and the sheet material 35 for forming the side sheet portion 34 are joined are formed at positions being apart from side edges of the absorbent member 33 on the side of the backsheet 32. The absorbent member 33 of the diaper 1A is structured with a lower absorbent core 331 being rectangular in plane view including a pair of lateral low stiffness portions (bending guide portions) 333 at the crotch section C, an upper absorbent core 332 which is smaller than the lower absorbent core 331 and is laminated onto the lower absorbent core 331, and a core wrap sheet (not illustrated) which covers the above. The low stiffness portions (bending guide portions) are scarcity portions having no structural material of the absorbent core such as pulp fibers, low basis weight portions having less structural material of the absorbent core than that of other portions, slits, or the like, and facilitate to raise both sides of the absorbent member 33 with decreased bending stiffness.

Similarly to the first embodiment, in the diaper 1A of the second embodiment, the stretchable region 34c of the side sheet portion 34 is formed so that the length Lm from the center line CL in the longitudinal direction of the diaper 1A (a line bisecting the entire length in the longitudinal direction of the diaper) to the front end portion 34m is longer than the length Lu from the center line CL in the longitudinal direction to the rear end portion 34u. Further, regarding the stretchable region 34c of the side sheet portion 34, at least a part of a portion located on the rear panel 2B is joined onto the rear panel 2B while the entire range in direction X of a portion located on the front panel 2A is not joined onto the front panel 2A.

Accordingly, the diaper 1A of the second embodiment can provide the same operational effects as the first embodiment.

In the above, the present invention is described based on the preferable embodiments. Here, not limited to the abovementioned embodiments, the present invention may be appropriately modified.

For example, the absorbent assembly 3 may not include the standing gather forming portion 51.

Further, the torso stretchable portion G2 may be formed over the entire width of the front panel 2A and/or the rear panel 2B. The extension stretchable portion G3 may be formed over the entire width of the front panel 2A and/or the rear panel 2B as well.

Further, although it is preferable that both the end portions 34m, 34u in the longitudinal direction of the stretchable region 34c reach onto the front panel 2A and the rear panel 2B, the end portion 34m on the side of the front panel may not reach the front body portion 20a. Further, the end portion 34u on the side of the rear panel may not reach the rear panel 2B or may reach the rear body portion 20b as traversing the rear extension portion 21b.

Further, the number of the elastic members 36 arranged at the side sheet portion 34 may be one, three or more (e.g., three to five) instead of two. The number of the elastic members 39 arranged at the standing gather forming portion 38 may be one as the second embodiment or three or more (e.g., three to five).

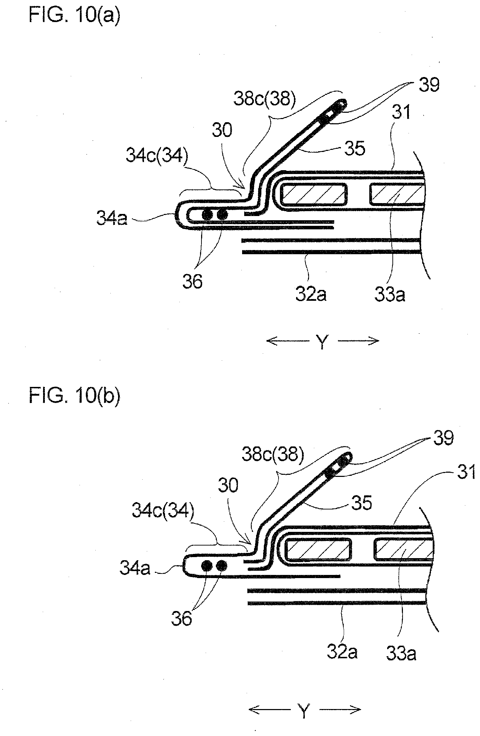

Further, the sheet laminated structure at the side sheet portion 34 is not necessarily a four-layer structure as the present embodiment. FIG. 10(a) illustrates a specific laminated structure of the side sheet portion 34 in the diapers 1, 1A of the embodiments. As described above, the standing gather 38c is structured with a two-layer laminated sheet with the sheet material 35 two-folded. Then, the side sheet portion 34 is structured with four sheet members 35 obtained by further two-folding the laminated sheet and the elastic members 36 are fixed between the two layers of laminated sheets.

In contrast, as illustrated in FIG. 10(b), the side sheet portion 34 may have a two-layer structure by extending only one sheet of the two sheet members 35 structuring the standing gather 38c outward in direction Y and performing two-folding along a folding portion arranged at the end part 34a at the free end side of the side gather. In this case, the elastic members 36 are fixed between the two sheet members 35 with adhesive. Further, the linear joint portion 30 is in a three-layer state having three sheet members 35 overlapped. The three layers are joined and integrated in a state that the topsheet 31 is interposed between one layer at the top side structuring the side sheet portion 34 and two layers structuring the standing gather 38c.

Further, although the side seal 4 is formed by joining the side edge portions 2a, 2b of the front and rear panels, it is also possible that a non-joint portion having a narrow width (e.g., more than 0 mm and 20 mm or less) where the front panel 2A and the rear panel 2B are not joined is formed outward of the side seal 4.

Further, other than a disposable pull-on diaper for an infant or an adult, the pull-on absorbent article may be an underwear-type sanitary napkin, or the like.

Portions which are not described in any one of the abovementioned embodiments and elements included in only one of the abovementioned embodiments may be appropriately applied respectively to other embodiments. Further, elements in the respective embodiments are appropriately replaceable among the embodiments from one another. In relation to the abovementioned embodiments, the present invention further discloses the following absorbent articles.

[1] A pull-on absorbent article having a waist opening and a pair of leg openings, comprising:
   a rectangular front panel adapted to be worn around a wearer's front;
   a rectangular rear panel adapted to be worn around a wearer's rear; and
   an absorbent assembly fixed to the front panel and the rear panel so as to bridge them,
   wherein each of the front panel and the rear panel includes
      a body portion which includes side seals located at both lateral sides of the panels; and
      an extension portion which is extended from the body portion toward a crotch portion and which has no side seals at both lateral sides of the panels,
   the absorbent assembly includes an absorbent member oblong in longitudinal direction and side sheet portions located outward of both side edges in the longitudinal direction of the absorbent member,
   the side sheet portion includes a stretchable region having stretchability due to an elastic member fixed to the side sheet portion in a stretched state, the stretchable region being located at the crotch portion and on the front panel and the rear panel,
   the stretchable region is formed in such a manner that a length from a center line in the longitudinal direction of the article to an end part of the stretchable region located on the side of the front panel is larger than a length from the center line in the longitudinal direction of the article to an end part of the stretchable region located on the side of the rear panel, and
   an entire range of a portion of the stretchable region located on the front panel is not joined onto the front panel and at least a part of the stretchable region located on the rear panel is joined onto the rear panel.
[2] The absorbent article according to subject [1], wherein "1.1Lu ≤ Lm ≤ 4.0Lu" is satisfied as Lm denoting the length of the stretchable region from the center line in the longitudinal direction of the absorbent article to the end part at the front panel side and Lu denoting the length of the stretchable region from the center line in the longitudinal direction of the absorbent article to the end part at the rear panel side.
[3] The absorbent article according to subject [1] or subject [2], wherein a length Lm is in a range of 52% to 80% of the total length (Lm + Lu) of the stretchable region preferably, in a range of 55% to 75% more preferably, and in a range of 55% to 70% even more preferably as Lm denoting the length of the stretchable region from the center line in the longitudinal direction of the absorbent article to the end part at the front panel side and Lu denoting the length of the stretchable region from the center line in the longitudinal direction of the absorbent article to the end part at the rear panel side, respectively.
[4] The absorbent article according to any one of subjects [1] to [3], wherein a length Lm is in a range of 25% to 40% of the total length L of the absorbent article preferably, and in a range of 28% to 38% more preferably as Lm denoting the length of the stretchable region from the center line in the longitudinal direction of the absorbent article to the end part at the front panel side and Lu denoting the length of the stretchable region from the center line in the longitudinal direction of the absorbent article to the end part at the rear panel side, respectively.
[5] The absorbent article according to any one of subjects [1] to [4], wherein the end part of the stretchable region at the front panel side does not reach the body portion.
[6] The absorbent article according to any one of subjects [1] to [5], wherein the side sheet portion is joined respectively onto the front panel and the rear panel on the side of an end part in the longitudinal direction of the absorbent assembly relative to the end part of the stretchable region.
[7] The pull-on absorbent article according to any one of subjects [1] to [6], wherein the end part of the stretchable region located on the side of the front panel is located on the body portion of the front panel, and
   the end part of the stretchable region located on the side of the rear panel is located on the extension portion of the rear panel.
[8] The pull-on absorbent article according to any one of subjects [1] to [7], wherein the absorbent member includes low-stiffness portions located at both sides in the longitudinal direction of the absorbent member, the low-stiffness portions extending along the longitudinal direction of the absorbent member.
[9] The pull-on absorbent article according to subject [8], wherein the end part of the stretchable region located on the side of the front panel extends from an end part of the low-stiffness portion located on the side of the front panel toward an edge of the waist opening at the front panel.
[10] The pull-on absorbent article according to any one of subjects [1] to [9], further comprising standing gathers located laterally inward of the side sheet portion in the both lateral sides of the absorbent assembly,
   wherein a stretchable region of the standing gather is extended from an end part of the stretchable region of the side sheet portion located on the side of the rear panel toward an edge of waist opening of the rear panel.
[11] The pull-on absorbent article according to any one of subjects [1] to [10], further comprising standing gathers located laterally inward of the side sheet portion in the both lateral sides of the absorbent assembly,
   wherein an end part of a stretchable region of the standing gather located on the side of the front panel does not reach a position of an end part of the stretchable region of the side sheet portion located on the side of the front panel.
[12] The pull-on absorbent article according to any one of subjects [1] to [11], wherein the absorbent member comprises an absorbent core and a core wrap sheet which covers the absorbent core,
   the absorbent core includes an aggregate of fibers, or an aggregate of fibers and particles of an absorbent polymer retained in the fibers, and
   a center line in the longitudinal direction of the absorbent core is located on the side of the front panel relative to the center line in the longitudinal direction of the article.
[13] The pull-on absorbent article according to any one of subjects [1] to [12], wherein each of the front panel and the rear panel comprising an outer layer sheet forming an outer face of the absorbent article, an inner layer sheet arranged on the side of the inner face of the outer layer sheet, and a plurality of thread-like elastic members arranged between the both sheets.
[14] The pull-on absorbent article according to subject [13], wherein the outer layer sheet and the inner layer sheet include folded extension portions, the folded extension portions being folded back on the side of the inner layer sheet at an edge portion which forms an opening circumferential edge of the waist opening.
[15] The pull-on absorbent article according to subject [14],
   wherein the folded extension portions are joined, at the side seal, to portions of the outer layer sheet and the inner layer sheet which are not folded back, and
   positions of the folded extension portions overlapped with the absorbent assembly in the longitudinal direction are joined to a surface of the absorbent assembly on the side of a topsheet with adhesive.
[16] The pull-on absorbent article according to any one of subjects [1] to [15],
   wherein a waist stretchable portion and a torso stretchable portion are formed at each of the body portion of the front panel and the body portion of the rear panel,
   an extension stretchable portion is formed at each of the extension portion of the front panel and the extension portion of the rear panel,
   the waist stretchable portion is formed, at each of the front panel and the rear panel, outward of the longitudinal end part of the absorbent assembly in the longitudinal direction of the absorbent article, and
   the torso stretchable portion is formed, at each of the front panel and the rear panel, between the waist stretchable portion and the extension portion in the longitudinal direction of the absorbent article.
[17] The pull-on absorbent article according to any one of subjects [1] to [15],
   wherein the side seal is formed by joining side edge portions of the front panel and the rear panel, and
   the side seal includes a non-joint portion located outward of the side seal and having a narrow width, the front panel and the rear panel being not joined at the non-joint portion.
[18] The pull-on absorbent article according to any one of subjects [1] to [17], wherein the absorbent article is for an infant.

### Industrial Applicability

A pull-on absorbent article of the present invention being an outer cover front-rear separation type is less likely to provide a strange feeling and an uncomfortable feeling at the crotch of a wearer as well as an uneasy feeling for leakage.

## Claims

1. A pull-on absorbent article having a waist opening and a pair of leg openings, comprising:
a rectangular front panel adapted to be worn around a wearer's front;
a rectangular rear panel adapted to be worn around a wearer's rear; and
an absorbent assembly fixed to the front panel and the rear panel so as to bridge them,
wherein each of the front panel and the rear panel includes
a body portion which includes side seals located at both lateral sides of the panels; and
an extension portion which is extended from the body portion toward a crotch portion and which has no side seals at both lateral sides of the panels,
the absorbent assembly includes an absorbent member oblong in longitudinal direction and side sheet portions located outward of both side edges in the longitudinal direction of the absorbent member,
the side sheet portion includes a stretchable region having stretchability due to an elastic member fixed to the side sheet portion in a stretched state, the stretchable region being located at the crotch portion and on the front panel and the rear panel,
the stretchable region is formed in such a manner that a length from a center line in the longitudinal direction of the article to an end part of the stretchable region located on the side of the front panel is larger than a length from the center line in the longitudinal direction of the article to an end part of the stretchable region located on the side of the rear panel, and
an entire range of a portion of the stretchable region located on the front panel is not joined onto the front panel and at least a part of the stretchable region located on the rear panel is joined onto the rear panel.

2. The pull-on absorbent article according to claim 1, wherein the end part of the stretchable region located on the side of the front panel does not reach the body portion.

3. The pull-on absorbent article according to claim 1 or claim 2, wherein the side sheet portion is joined respectively onto the front panel and the rear panel on the side of a longitudinal end part of the absorbent assembly relative to the end part of the stretchable region.

4. The pull-on absorbent article according to claim 1,
wherein the end part of the stretchable region located on the side of the front panel is located on the body portion of the front panel, and
the end part of the stretchable region located on the side of the rear panel is located on the extension portion of the rear panel.

5. The pull-on absorbent article according to any one of claims 1 to 4, wherein the absorbent member includes low-stiffness portions located at both sides in the longitudinal direction of the absorbent member, the low-stiffness portions extending along the longitudinal direction of the absorbent member.

6. The pull-on absorbent article according to claim 5, wherein the end part of the stretchable region located on the side of the front panel extends from an end part of the low-stiffness portion located on the side of the front panel toward an edge of the waist opening at the front panel.

7. The pull-on absorbent article according to any one of claims 1 to 6, further comprising standing gathers located laterally inward of the side sheet portion in the both lateral sides of the absorbent assembly,
wherein a stretchable region of the standing gather is extended from an end part of the stretchable region of the side sheet portion located on the side of the rear panel toward an edge of waist opening of the rear panel.

8. The pull-on absorbent article according to any one of claims 1 to 7, further comprising standing gathers located laterally inward of the side sheet portion in the both lateral sides of the absorbent assembly,
wherein an end part of a stretchable region of the standing gather located on the side of the front panel does not reach a position of an end part of the stretchable region of the side sheet portion located on the side of the front panel.

9. The pull-on absorbent article according to any one of claims 1 to 8,
wherein the absorbent member comprises an absorbent core and a core wrap sheet which covers the absorbent core,
the absorbent core includes an aggregate of fibers, or an aggregate of fibers and particles of an absorbent polymer retained in the fibers, and
a center line in the longitudinal direction of the absorbent core is located on the side of the front panel relative to the center line in the longitudinal direction of the article.

10. The pull-on absorbent article according to any one of claims 1 to 9, wherein each of the front panel and the rear panel comprises an outer layer sheet forming an outer face of the absorbent article, an inner layer sheet arranged on the side of the inner face of the outer layer sheet, and a plurality of thread-like elastic members arranged between the both sheets.

11. The pull-on absorbent article according to claim 10, wherein the outer layer sheet and the inner layer sheet include folded extension portions, the folded extension portions being folded back on the side of the inner layer sheet at an edge portion which forms an opening circumferential edge of the waist opening.

12. The pull-on absorbent article according to claim 11,
wherein the folded extension portions are joined, at the side seal, to portions of the outer layer sheet and the inner layer sheet which are not folded back, and
positions of the folded extension portions overlapped with the absorbent assembly in the longitudinal direction are joined to a surface of the absorbent assembly on the side of a topsheet with adhesive.

13. The pull-on absorbent article according to any one of claims 1 to 12,
wherein a waist stretchable portion and a torso stretchable portion are formed at each of the body portion of the front panel and the body portion of the rear panel,
an extension stretchable portion is formed at each of the extension portion of the front panel and the extension portion of the rear panel,
the waist stretchable portion is formed, at each of the front panel and the rear panel, outward of the longitudinal end part of the absorbent assembly in the longitudinal direction of the absorbent article, and
the torso stretchable portion is formed, at each of the front panel and the rear panel, between the waist stretchable portion and the extension portion in the longitudinal direction of the absorbent article.

14. The pull-on absorbent article according to any one of claims 1 to 13,
wherein the side seal is formed by joining side edge portions of the front panel and the rear panel, and
the side seal includes a non-joint portion located outward of the side seal and having a narrow width, the front panel and the rear panel being not joined at the non-joint portion.

15. The pull-on absorbent article according to any one of claims 1 to 14, wherein the absorbent article is for an infant.

## Patentansprüche

1. Absorbierender Artikel vom Höschen-Typ mit einer Taillenöffnung und zwei Beinöffnungen, aufweisend:
einen rechteckigen Vorderstreifen, der beim Tragen um die Vorderseite eines Trägers herum angeordnet ist;
einen rechteckigen Rückstreifen, der beim Tragen um die Rückseite eines Trägers herum angeordnet ist; und
eine absorbierende Anordnung, die an dem Vorderstreifen und dem Rückstreifen befestigt ist, um sie brückenartig zu verbinden,
wobei sowohl der Vorderstreifen als auch der Rückstreifen aufweist:
einen Körperabschnitt, der Seitenversiegelungen an beiden lateralen Seiten der Streifen aufweist; und
einen Erweiterungsabschnitt, der sich von dem Körperabschnitt hin zu einem Schrittabschnitt erstreckt und der keine Seitenversiegelungen an beiden lateralen Seiten der Streifen hat,
wobei die absorbierende Anordnung - in Längsrichtung betrachtet - ein längliches absorbierendes Element und außerhalb von beiden Längsseitenkanten des absorbierenden Elements angeordnete Seitenschichtabschnitte aufweist,
der Seitenschichtabschnitt einen dehnbaren Bereich aufweist, der aufgrund eines in einem gedehnten Zustand an dem Seitenschichtabschnitt befestigten elastischen Elements eine Dehnbarkeit hat, wobei sich der dehnbare Bereich am Schrittabschnitt und am Vorderstreifen und am Rückstreifen befindet,
der dehnbare Bereich auf eine solche Weise gebildet ist, dass eine Länge, die sich von einer - in Längsrichtung betrachtet - Mittellinie des Artikels zu einem vorderstreifenseitigen Endabschnitt des dehnbaren Bereichs erstreckt, größer ist als eine Länge, die sich von einer - in Längsrichtung betrachtet - Mittellinie des Artikels zu einem rückstreifenseitigen Endabschnitt des dehnbaren Bereichs erstreckt, und
ein gesamtes Gebiet des am Vorderstreifen angeordneten Teils des dehnbaren Bereichs nicht mit dem Vorderstreifen verbunden ist und mindestens ein Teil des am Rückstreifen angeordneten dehnbaren Bereichs mit dem Rückstreifen verbunden ist.

2. Absorbierender Artikel vom Höschen-Typ nach Anspruch 1, wobei der vorderstreifenseitige Endabschnitt des dehnbaren Bereichs den Körperabschnitt nicht erreicht.

3. Absorbierender Artikel vom Höschen-Typ nach Anspruch 1 oder Anspruch 2, wobei der Seitenschichtabschnitt mit dem Vorderstreifen bzw. dem Rückstreifen verbunden ist auf der Seite eines Längsendabschnitts der absorbierenden Anordnung relativ zu dem Endabschnitt des dehnbaren Bereichs.

4. Absorbierender Artikel vom Höschen-Typ nach Anspruch 1, wobei
der vorderstreifenseitige Endabschnitt des dehnbaren Bereichs an dem Körperabschnitt des Vorderstreifens angeordnet ist, und
der rückstreifenseitige Endabschnitt des dehnbaren Bereichs an dem Erweiterungsabschnitt des Rückstreifens angeordnet ist.

5. Absorbierender Artikel vom Höschen-Typ nach einem der Ansprüche 1 bis 4, wobei das absorbierende Element Abschnitte geringer Steifheit aufweist, die an beiden Längsseiten des absorbierenden Elements angeordnet sind und sich entlang der Längsrichtung des absorbierenden Elements erstrecken.

6. Absorbierender Artikel vom Höschen-Typ nach Anspruch 5, wobei der vorderstreifenseitige Endabschnitt des dehnbaren Bereichs sich von einem vorderstreifenseitigen Endabschnitt des Abschnitts geringer Steifheit hin zu einer Kante der Taillenöffnung an dem Vorderstreifen erstreckt.

7. Absorbierender Artikel vom Höschen-Typ nach einem der Ansprüche 1 bis 6, ferner aufweisend stehende Kräusel, die lateral einwärts des Seitenschichtabschnitts in den beiden lateralen Seiten der absorbierenden Anordnung angeordnet sind,
wobei sich ein dehnbarer Bereich der stehenden Kräusel von einem rückstreifenseitigen Endabschnitt des dehnbaren Bereichs des Seitenschichtabschnitts hin zu einer Kante der Taillenöffnung des Rückstreifens erstreckt.

8. Absorbierender Artikel vom Höschen-Typ nach einem der Ansprüche 1 bis 7, ferner aufweisend stehende Kräusel, die lateral einwärts des Seitenschichtabschnitts in den beiden lateralen Seiten der absorbierenden Anordnung angeordnet sind,
wobei ein vorderstreifenseitiger Endabschnitt des dehnbaren Bereichs der stehenden Kräusel nicht bis zu einer Position eines vorderstreifenseitigen Endabschnitts des dehnbaren Bereichs des Seitenschichtabschnitts reicht.

9. Absorbierender Artikel vom Höschen-Typ nach einem der Ansprüche 1 bis 8, wobei
das absorbierende Element einen absorbierenden Kern und eine Kernhüllschicht aufweist, die den Kern umgibt,
der absorbierende Kern ein Faseraggregat oder ein Faseraggregat und in den Fasern eingeschlossene Partikel eines absorbierenden Polymers aufweist, und
eine - in Längsrichtung betrachtete - Mittellinie des absorbierenden Kerns relativ zu der - in Längsrichtung betrachteten - Mittellinie des Artikels zur Seite des Vorderstreifens hin versetzt angeordnet ist.

10. Absorbierender Artikel vom Höschen-Typ nach einem der Ansprüche 1 bis 9, wobei sowohl der Vorderstreifen als auch der Rückstreifen eine Außenschicht, die eine Außenfläche des absorbierenden Artikels bildet, eine an der Seite der Innenfläche der Außenschicht angeordnete Innenschicht und mehrere zwischen beiden Schichten angeordnete fadenförmige elastische Elemente aufweisen.

11. Absorbierender Artikel vom Höschen-Typ nach Anspruch 10, wobei die Außenschicht und die Innenschicht gefaltete Erweiterungsabschnitte aufweisen, wobei die gefalteten Erweiterungsabschnitte an einem Kantenabschnitt, der eine Öffnungsumfangskante der Taillenöffnung bildet, auf Seite der Innenschicht zurückgefaltet sind.

12. Absorbierender Artikel vom Höschen-Typ nach Anspruch 11, wobei
die gefalteten Erweiterungsabschnitte an der Seitenversiegelung mit Abschnitten der Außenschicht und der Innenschicht, die nicht zurückgefaltet sind, verbunden sind, und
Positionen der gefalteten Erweiterungsabschnitte, die mit der absorbierenden Anordnung in Längsrichtung überlappen, mit Klebstoff an einer Oberfläche der absorbierenden Anordnung auf der Seite einer Oberschicht angebracht sind.

13. Absorbierender Artikel vom Höschen-Typ nach einem der Ansprüche 1 bis 12, wobei
sowohl am Körperabschnitt des Vorderstreifens als auch am Körperabschnitt des Rückstreifens ein dehnbarer Taillenabschnitt und ein dehnbarer Rumpfabschnitt gebildet sind,
sowohl am Erweiterungsabschnitt des Vorderstreifens als auch am Erweiterungsabschnitt des Rückstreifens ein dehnbarer Erweiterungsabschnitt gebildet ist,
sowohl am Vorderstreifen als auch am Rückstreifen der dehnbare Taillenabschnitt - in Längsrichtung betrachtet - außerhalb des Längsendeabschnitts der absorbierenden Anordnung des absorbierenden Artikels gebildet ist, und
sowohl am Vorderstreifen als auch am Rückstreifen der dehnbare Rumpfabschnitt - in Längsrichtung betrachtet - zwischen dem dehnbaren Taillenabschnitt und dem Erweiterungsabschnitt des absorbierenden Artikels gebildet ist.

14. Absorbierender Artikel vom Höschen-Typ nach einem der Ansprüche 1 bis 13, wobei
die Seitenversiegelung durch Verbinden von Seitenkantenabschnitten des Vorderstreifens und des Rückstreifens gebildet ist und
die Seitenversiegelung einen nichtverbundenen Abschnitt aufweist, der außerhalb der Seitenversiegelung angeordnet ist und eine schmale Breite hat, wobei der Vorderstreifen und der Rückstreifen an dem nichtverbundenen Abschnitt nicht verbunden sind.

15. Absorbierender Artikel vom Höschen-Typ nach einem der Ansprüche 1 bis 14, wobei der absorbierende Artikel für ein Kleinkind ist.

## Revendications

1. Article absorbant à enfiler ayant une ouverture de taille et une paire d'ouvertures de jambe, comprenant :
un panneau avant rectangulaire adapté pour être porté autour de la partie avant de l'utilisateur ;
un panneau arrière rectangulaire adapté pour être porté autour de la partie arrière de l'utilisateur ; et
un ensemble absorbant fixé sur le panneau avant et le panneau arrière afin de les relier,
dans lequel chacun parmi le panneau avant et le panneau arrière comprend :
une partie de corps qui comprend des joints d'étanchéité latéraux positionnés des deux côtés latéraux des panneaux ; et
une partie d'extension qui est étendue à partir de la partie de corps vers une partie d'entrejambes et qui n'a pas de joints d'étanchéité latéraux des deux côtés latéraux des panneaux,
l'ensemble absorbant comprend un élément absorbant oblong dans la direction longitudinale et des parties de feuille latérales positionnées vers l'extérieur des deux bords latéraux dans la direction longitudinale de l'élément absorbant,
la partie de feuille latérale comprend une région extensible ayant une extensibilité due à un élément élastique fixé sur la partie de feuille latérale dans un état étiré, la région extensible étant positionnée au niveau de la partie d'entrejambes et sur le panneau avant et le panneau arrière,
la région extensible est formée de sorte qu'une longueur allant d'une ligne centrale dans la direction longitudinale de l'article à une partie d'extrémité de la région extensible positionnée sur le côté du panneau avant est plus grande qu'une longueur allant de la ligne centrale dans la direction longitudinale de l'article à une partie d'extrémité de la région extensible positionnée sur le côté du panneau arrière, et
une plage entière d'une partie de la région extensible positionnée sur le panneau avant n'est pas assemblée sur le panneau avant et au moins une partie de la région extensible positionnée sur le panneau arrière est assemblée sur le panneau arrière.

2. Article absorbant à enfiler selon la revendication 1, dans lequel la partie d'extrémité de la région extensible positionnée sur le côté du panneau avant n'atteint pas la partie de corps.

3. Article absorbant à enfiler selon la revendication 1 ou la revendication 2, dans lequel la partie de feuille latérale est assemblée respectivement sur le panneau avant et le panneau arrière sur le côté d'une partie d'extrémité longitudinale de l'ensemble absorbant par rapport à la partie d'extrémité de la région extensible.

4. Article absorbant à enfiler selon la revendication 1,
dans lequel la partie d'extrémité de la région extensible positionnée sur le côté du panneau avant est positionnée sur la partie de corps du panneau avant, et
la partie d'extrémité de la région extensible positionnée sur le côté du panneau arrière est positionnée sur la partie d'extension du panneau arrière.

5. Article absorbant à enfiler selon l'une des revendications 1 à 4, dans lequel l'élément absorbant comprend des parties à faible rigidité positionnées des deux côtés dans la direction longitudinale de l'élément absorbant, les parties à faible rigidité s'étendant le long de la direction longitudinale de l'élément absorbant.

6. Article absorbant à enfiler selon la revendication 5, dans lequel la partie d'extrémité de la région extensible positionnée sur le côté du panneau avant s'étend à partir d'une partie d'extrémité de la partie à faible rigidité positionnée sur le côté du panneau avant vers un bord de l'ouverture de taille au niveau du panneau avant.

7. Article absorbant à enfiler selon l'une des revendications 1 à 6, comprenant en outre des fronces droites positionnées latéralement vers l'intérieur de la partie de feuille latérale des deux côtés latéraux de l'ensemble absorbant,
dans lequel une région extensible de la fronce droite est étendue à partir d'une partie d'extrémité de la région extensible de la partie de feuille latérale positionnée sur le côté du panneau arrière vers un bord de l'ouverture de taille du panneau arrière.

8. Article absorbant à enfiler selon l'une des revendications 1 à 7, comprenant en outre des fronces droites positionnées latéralement vers l'intérieur de la partie de feuille latérale des deux côtés latéraux de l'ensemble absorbant,
dans lequel une partie d'extrémité d'une région extensible de la fronce droite positionnée sur le côté du panneau avant n'atteint pas une position d'une partie d'extrémité de la région extensible de la partie de feuille latérale positionnée sur le côté du panneau avant.

9. Article absorbant à enfiler selon l'une des revendications 1 à 8,
dans lequel l'élément absorbant comprend un coeur absorbant et une feuille d'enroulement de coeur qui recouvre le coeur absorbant,
le coeur absorbant comprend un agrégat de fibres, ou un agrégat de fibres et de particules d'un polymère absorbant retenu dans les fibres, et
une ligne centrale dans la direction longitudinale du coeur absorbant est positionnée sur le côté du panneau avant par rapport à la ligne centrale dans la direction longitudinale de l'article.

10. Article absorbant à enfiler selon l'une des revendications 1 à 9, dans lequel chacun parmi le panneau avant et le panneau arrière comprend une feuille de couche externe formant une face externe de l'article absorbant, une feuille de couche interne agencée sur le côté de la face interne de la feuille de couche externe, et une pluralité d'éléments élastiques en forme de fil agencés entre les deux feuilles.

11. Article absorbant à enfiler selon la revendication 10, dans lequel la feuille de couche externe et la feuille de couche interne comprennent des parties d'extension pliées, les parties d'extension pliées étant repliées sur le côté de la feuille de couche interne au niveau d'une partie de bord qui forme un bord circonférentiel d'ouverture de l'ouverture de taille.

12. Article absorbant à enfiler selon la revendication 11,
dans lequel les parties d'extension pliées sont assemblées, au niveau du joint d'étanchéité latéral, aux parties de la feuille de couche externe et de la feuille de couche interne qui ne sont pas repliées, et
les positions des parties d'extension pliées recouvertes avec l'ensemble absorbant dans la direction longitudinale sont assemblées à une surface de l'ensemble absorbant sur le côté d'une feuille supérieure avec de l'adhésif.

13. Article absorbant à enfiler selon l'une des revendications 1 à 12,
dans lequel une partie extensible de taille et une partie extensible de torse sont formées au niveau de chacune parmi la partie de corps du panneau avant et la partie de corps du panneau arrière,
une partie extensible d'extension est formée au niveau de chacune parmi la partie d'extension du panneau avant et la partie d'extension du panneau arrière,
la partie extensible de taille est formée, au niveau de chacun parmi le panneau avant et le panneau arrière, vers l'extérieur de la partie d'extrémité longitudinale de l'ensemble absorbant dans la direction longitudinale de l'article absorbant, et
la partie extensible de torse est formée, au niveau de chacun parmi le panneau avant et le panneau arrière, entre la partie extensible de taille et la partie d'extension dans la direction longitudinale de l'article absorbant.

14. Article absorbant à enfiler selon l'une des revendications 1 à 13,
dans lequel le joint d'étanchéité latéral est formé en assemblant les parties de bord latérales du panneau avant et du panneau arrière, et
le joint d'étanchéité latéral comprend une partie sans joint positionnée vers l'extérieur du joint d'étanchéité latéral et ayant une largeur étroite, le panneau avant et le panneau arrière n'étant pas assemblés au niveau de la partie sans joint.

15. Article absorbant à enfiler selon l'une des revendications 1 à 14, dans lequel l'article absorbant est prévu pour un enfant en bas âge.
